# EUROPÄISCHE PATENTSCHRIFT

(11) **EP 2 151 256 B1**
(45) Veröffentlichungstag und Bekanntmachung des Hinweises auf die Patenterteilung: **10.12.2014**
(21) Anmeldenummer: 09163907.0
(22) Anmeldetag: 26.06.2009
(51) Int. Cl.: A61L 31/08, A61L 31/02

(54) **Endoprothese und Verfahren zur Herstellung derselben**
Endoprosthesis and method for manufacturing the same
Endoprothèse et son procédé de fabrication

(30) Priorität: 28.07.2008 DE 102008040791
(43) Veröffentlichungstag der Anmeldung: 10.02.2010
(73) Patentinhaber: Biotronik VI Patent AG, 6341 Baar (CH)
(72) Erfinder: Rohde, Roland, 31303, Burgdorf (DE)
(74) Vertreter: Lindner-Vogt, Karin L.

(56) Entgegenhaltungen:
- WO-A1-2007/147246
- DE-A1-102004 043 231

## Beschreibung

Die Erfindung betrifft eine intraluminale Endoprothese, vorzugsweise einen Stent, bestehend aus einem Grundkörper und ggf. einer die Oberfläche des Grundkörpers mindestens teilweise bedeckenden Beschichtung sowie ein Verfahren zur Herstellung einer derartigen Endoprothese.

Endoprothesen sind Prothesen und Implantate, welche dauerhaft im Körper verbleiben. Ein besonders wichtiger Vertreter der intraluminalen Endoprothesen sind Stents (Gefäßstützen). Stents sind endovaskuläre Prothesen, welche zur Behandlung von Stenosen (Gefäßverengungen) eingesetzt werden können. Sie weisen einen rohrförmigen oder hohlzylinderförmigen Grundkörper auf, der vorzugsweise aus zick-zack- oder mäanderförmig gefalteten, im Wesentlichen in Umfangsrichtung verlaufenden Stegen als Stützelemente und weiterhin aus diese Stützelemente verbindenden, in Längsrichtung verlaufenden Stegen als Verbindungsstreben besteht. Der Grundkörper kann mindestens teilweise mit einer Beschichtung versehen sein und ist an beiden Enden in Richtung der Achse des Hohlzylinders offen. Eine derartige Endoprothese wird beispielsweise mittels eines Katheters in das zu behandelnde Gefäß eingesetzt und dient dazu, das Gefäß zu stützen. Durch den Einsatz von Stents können verengte Bereiche in den Gefäßen erweitert werden, so dass ein Lumengewinn resultiert.

Nach dem Einsetzen eines Stents in ein Gefäß besteht die Gefahr einer Restenose, d. h. einer Wiederverengung des mit dem Stent behandelten Gefäßbereichs auf Grund verschiedener Prozesse, beispielsweise einer Koagulation der Körperflüssigkeit oder Okklusion in diesem Bereich infolge einer Neointimabildung, infolge einer Strömungsveränderung oder infolge eines Entzündungsprozesses.

Eine Möglichkeit zur Lösung dieses Problems besteht darin, den Stent aus einem biodegradierbaren Werkstoff zu fertigen. Unter Biodegradation werden hydrolytische, enzymatische und andere stoffwechselbedingte Abbauprozesse im lebenden Organismus (in vivo) verstanden, die vor allem durch die mit der Endoprothese in Kontakt gelangenden Körperflüssigkeiten verursacht werden und zu einer allmählichen Auflösung zumindest großer Teile der Endoprothese führen. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für den Grundkörper biodegradierbarer Endoprothesen geeignete Werkstoffe können beispielsweise polymerer oder metallischer Natur sein.

Aus der Druckschrift EP 1 270 023 B1 ist eine solche Gefäßstütze aus einem durch Korrosion in vivo abbaubarem metallischen Werkstoff bekannt, der aus einer Legierung mit dem Hauptbestandteil Magnesium gebildet wird. Der Werkstoff umfasst insbesondere 79 - 97% Magnesium, 2 - 5% Aluminium, 0 - 12% Lithium und 1 - 4% Seltene Erden, insbesondere Cer, Lanthan, Neodym und/oder Praseodym. Magnesium ist physiologisch sehr gut verträglich und durch die geeignete Wahl der übrigen Legierungsbestandteile kann die zu erwartende Abbaugeschwindigkeit gesteuert werden. Aufgrund von Materialinhomogenitäten kann es jedoch gerade bei unedlen Metallen wie Magnesium zu einer lokal beschleunigten Korrosion kommen, die einen vorzeitigen mechanischen Integritäts- und Stabilitätsverlust des Implantats zur Folge hat. Ferner kann es auf Implantatoberflächen zu bakterieller Besiedelung kommen, die als Infektionsquelle und im Falle von Stents auch zur Störung von Gerinnungsprozessen, insbesondere zu Fibrinablagerungen und Thrombosen führen kann. Ein weiterer Nachteil insbesondere von Implantaten aus Leichtmetallen ist die fehlende Röntgensichtbarkeit, die eine effektive Verfolgung und Nachkontrolle des Implantationsprozesses verhindert.

Aus der Druckschrift DE 103 08 186 B4 ist ein antimikrobiell wirkendes Phosphatglas mit einer Vielzahl von Bestandteilen, insbesondere 66 - 80 Gew.% P₂O₅ bekannt. Die Glaszusammensetzung weist eine hohe chemische Beständigkeit sowie eine hohe Reaktivität auf und kann in dem Bereich der Medizin und Kosmetik angewendet werden. Die in der Druckschrift angegebene Zusammensetzung ist jedoch als Grundkörper- oder Beschichtungsmaterial für intraluminale Endoprothesen nicht geeignet, da sie z.B. mit Al₂O₃ sowie den angegebenen Cu-, Ge-, Te-, Cr- und F-Verbindungen toxische oder potentiell toxische Bestandteile aufweist.

Ein weiteres Problem eines gestenteten Gefäßes besteht darin, dass abbaubare, d.h. biodegradierbare Stents aufgrund zu schneller initialer Degradation einen zu frühen Integritätsverlust erleiden können. Ferner neigen derartige medizinische Implantate aufgrund ihrer Einsatzweise oder Patientengruppen-bezogener Prädisposition zur bakteriellen Besiedelung und entsprechender Gefährdung des Patienten und einer sich hieraus ergebenen Notwendigkeit einer Re-Operation/Antibiotikabehandlung. Zudem ist es wünschenswert, wenn Stents oder andere intraluminale Endoprothesen radiologisch sichtbar sind, so dass beim Einbringen oder im Verlauf der weiteren Behandlung das Implantat lokalisiert werden kann.

Die Aufgabe der vorliegenden Erfindung besteht demnach darin, eine intraluminale Endoprothese zu schaffen, welche eine Wiederverengung des gestenteten Gefäßes durch Neointimabildung verhindert und bei der die Integrität über einen längeren Zeitraum gegeben ist. Die Aufgabe besteht ferner darin, ein Verfahren zur Herstellung einer intraluminalen Endoprothese anzugeben, das einfach und kostengünstig ist.

Die vorstehend genannte Aufgabe wird durch eine intraluminale Endoprothese gelöst, deren Grundkörper und/oder deren die Oberfläche des Grundkörpers mindestens teilweise bedeckende Beschichtung mindestens eine Verbindung aus der Gruppe Polyphosphate, Magnesiumoxidhalogenide, vorzugsweise Sorelzement, aufweist.

Die genannten Verbindungen erfüllen die oben angegebene Aufgabenstellung.

Hierbei besteht der Vorteil einer erfindungsgemäßen intraluminalen Endoprothese mit Polyphosphaten darin, dass Polyphosphate als Bestandteil des Grundkörpers bzw. einer Beschichtung durch rechtzeitige Biodegradation des Grundkörpers die Restenose verhindern. Als Beschichtung eines abbaubaren Stents, beispielsweise eines AMS-Stents (absorbierbarer Metallstent), verhindert eine Beschichtung mit Polyphosphaten durch Verlangsamung des Wasserzutritts und im Falle eines Magnesium-Stents durch eine aktive Beeinflussung des Oberflächen-pH-Wertes den Degradationsverlauf. Ferner sind Polyphosphate keimhemmend.

Bei intraluminalen Endoprothesen mit einem Grundkörper oder einer Beschichtung enthaltend Magnesiumoxidhalogeniden (auch Magnesiumoxyhalogenid genannt), vorzugsweise Sorelzement (auch als Magnesia-Zement, Sorels Zement oder Magnesitbinder bezeichnet, Zusammensetzung: MgCl₂ x 3Mg(OH)₂ x 8H₂O) kann ebenfalls eine Restenose durch rechtzeitige Biodegradation des Stentkörpers verhindert werden.

Insbesondere ist es vorteilhaft, dass sich die mechanischen Eigenschaften von Sorelzement und anderen Magnesiumoxidhalogeniden bzw. Polyphosphaten durch Zuschläge steuern lassen. Als Zuschläge zur Steuerung mechanischer Eigenschaften eignen sich insbesondere Proteine wie Gelatine, Albumin und Fibrin; Polymere wie Polyphosphate, Polylaktate, Polyhydroxybuttersäure, Hyaluronsäure, Cellulose und andere Polysaccharide, jodhaltige Kontrastmittel; Phosphate, Jod. Der Masseanteil der Zuschläge kann dabei bis zu 50% des resultierenden Verbunds ausmachen, bei grobkörnigen Zuschlägen bis zu 80%.

Sorelzement enthält in vorteilhafter Weise lediglich Bestandteile, die im Blut in physiologischer Weise vorkommen, so dass Sorelzement biokompatibel ist. Sorelzement haftet auf metallischen und vielen anderen Oberflächen, verhindert die Wasserdiffusion, wird in wässrigen Lösungen abgebaut und ist deshalb als Beschichtung besonders gut geeignet. Durch eine nachträgliche Behandlung mit Phosphorsäure oder löslichen Phosphaten (Phosphatieren) lässt sich zudem der Abbau von Sorelzement verlangsamen. Bei Zugabe hydrophober Beimengungen, z.B. hydrophobe Polymere, PTFE, Fettsäureester, Cholesterinester, Wachse, Phosphatester und/oder Kieselsäureethylester, kann außerdem die Hydrophilie und damit die Wasserlöslichkeit reduziert werden.

Eine bevorzugte Zusammensetzung einer Grundkörpers und/oder einer Beschichtung mit Polyphosphaten und ggf. auch Magnesiumoxidhalogeniden enthält vorzugsweise die folgenden Bestandteile (alle nachfolgenden %-Angaben ohne Bezug zu einer anderen Größe sind Gew.%):
P₂O₅ etwa 30-100%, ZnO etwa < 1%, Alkali-Verbindungen etwa > 1%, Na₂O etwa 0-5%, K₂O etwa 0-15%, Li₂O etwa 0-15%, AgJ etwa 0-15%, MgJ₂ etwa 0-20%, MgCl₂ etwa 0-30%, MgO etwa 0-60%, Al₂O₃ etwa < 1%. Die vorstehend angegebene Zusammensetzung bezieht sich auf den wasserfreien Mineralgehalt.

Außerdem ist es von Vorteil, die Stöchiometrie der Bestandteile MgO (bzw. Mg(OH)₂) und MgCl₂ des Sorelzements des Grundkörpers und/oder der Beschichtung so zu verändern, dass sich ein MgO-Unter- oder-Überschuss ausbildet. Dadurch lassen sich GrenzflächenpH-Werte und Abbauraten von Verbundmaterialien über den Einfluss der Zuschläge hinaus zusätzlich steuern. Besonders vorteilhaft ist in diesem Zusammenhang ein MgCl₂ : Mg(OH)₂- Verhältnis von etwa 1:1 bis 1:7, insbesondere von etwa 1:3 bis 1:6.

Für die Verwendung als röntgenopakes Material in dem Grundkörper oder der Beschichtung kann MgCl₂ des Sorelzements ganz oder teilweise durch MgJ₂ ersetzt werden, so dass die Summenformel lautet:

[(1-a) MgCl₂ a MgJ₂] x b Mg(OH)₂ x c H₂O mit 0 < a ≤ 1, 1 < b ≤ 7, c > 1. (Formel 1)

In einem bevorzugten Ausführungsbeispiel ist der Grundkörper und/oder die Beschichtung mit mindestens einer pharmazeutisch aktiven Substanz und/oder einer röntgenopaken (radioopaken) Substanz, vorzugsweise mindestens einem Element oder einer Verbindung der Gruppe
Biopolymere, vorzugsweise Proteine, Peptide, Aminosäuren, Nucleinsäuren, Polysaccharide,
Lipide, vorzugsweise Fettsäuren, Fettsäureester, Wachse,
hydrophobe Verbindungen, vorzugsweise Cholesterinester, Phosphatester, Kieselsäureethylester,
röntgenopake Elemente oder Verbindungen, vorzugsweise Edelmetalle, Metallsalze, Jodverbindungen,
versehen.

Hierbei ist eine "pharmazeutisch aktive Substanz" (oder therapeutisch oder pharmakologisch aktive oder wirksame Substanz) im Sinne der Erfindung ein pflanzlicher, tierischer oder synthetisierter Wirkstoff (Medikament), der in geeigneter Dosierung als Therapeutikum zur Beeinflussung von Zuständen oder Funktionen des Körpers, als Ersatz für natürlich vom menschlichen oder tierischen Körper erzeugte Wirkstoffe sowie zur Beseitigung oder zum Unschädlichmachen von Krankheitserregern oder Körperfremdstoffen Einsatz findet. Die Freisetzung der Substanz in der Endoprothesenumgebung hat einen positiven Effekt auf den Heilungsverlauf bzw. wirkt pathologischen Veränderungen des Gewebes infolge eines chirurgischen Eingriffes entgegen.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können in weiteren Ausführungsbeispielen aus einer oder mehreren Substanzen der Wirkstoffgruppe der oben angegebenen Verbindungen oder Elemente oder der Kalziumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorika (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Peptide, der Vasodilatatoren (wie beispielsweise Sartane), der antiproliferativ wirkenden Stoffe der Taxole oder Taxane, hier vorzugsweise Paclitaxel oder Sirolimus und seine Derivate, sowie aus solchen lipophilen Substanzen, die eine Gewebekalzifizierung oder die Neointimabildung hemmen, wie Vitamin A- und D-Derivate und Phyllochinon/Menachinon- (Vitamin K)-Derivate bestehen.

In eine Sorelzementmatrix können pharmazeutisch aktive Substanzen dadurch eingebracht werden, dass bei der Herstellung der Sorelzementmatrix in einer oder beiden Sorelzementkomponenten (trockenes oder aufgeschwemmtes MgO/ Mg(OH)₂ und trockenes oder gelöstes MgCl*6H₂O) eine oder mehrere Lösungen oder Aufschwemmungen der einzubringenden Zuschläge zugefügt sind. Insbesondere können vor der Herstellung des Zements grobkörnige Zuschläge, beispielsweise die pharmazeutisch aktive Substanz und/oder die radioopake Substanz tragende Polymerkügelchen oder Proteinglobuli, mit einer der Sorel-Komponenten vermischt oder an dem Stentgrundkörper fixiert werden, bevor die Sorelmatrix aufgebracht wird.

In einem weiteren bevorzugten Ausführungsbeispiel weist die Endoprothese einen Grundkörper, der eine Metalllegierung und/oder ein Polymer und/oder ein Polyphosphat und/oder eine Sorelzement enthält, sowie eine Beschichtung auf, die Sorelzement enthält, wobei die Beschichtung eine Schichtdicke von mindestens etwa 5 µm, vorzugsweise mindestens etwa 50 µm, maximal etwa 200 µm hat. Die Sorelzement-Beschichtung erzeugt bei geeigneter MgCl₂:Mg(OH)₂-Stöchiometrie ein alkalisches Milieu, das den Korrosionsangriff durch die Körperflüssigkeiten auf den tragenden Grundkörper reduziert.

Besonders bevorzugt lassen sich die chemischen/physikalischen Eigenschaften und die Degradation dadurch steuern, dass die Endoprothese mindestens teilweise mit einer Polyphosphat-Beschichtung versehen ist, die eine Schichtdicke von mindestens 5µm, vorzugsweise mindestens etwa 30 µm, maximal etwa 200 µm hat, wobei die Moleküle der Polyphosphat-Beschichtung vorzugsweise eine Kettenlänge von mindestens zehn Phosphatgruppen aufweisen.

In einem weiteren Ausführungsbeispiel wird ein biodegradierbarer Metallstent, vorzugsweise aus einer Magnesiumlegierung, mit einer Polyphosphatschicht mit einem P₂O₅-Gehalt von 85 Gew.% sowie 5 Gew.% Na₂O, 5 Gew.% AgJ, 5 Gew.% MgO, bezogen auf den wasserfreien Mineralgehalt, in einer Schichtdicke von mindestens etwa 30 µm versehen und dann mit einer MgJ₂ - Lösung gespült. Bei der Spülung findet ein Austausch von Na-Ionen statt.

Besonders kostengünstig ist die Herstellung einer intraluminalen Endoprothese, bei der die Beschichtung enthaltend mindestens eine Verbindung aus der Gruppe Polyphosphate, Magnesiumoxidhalogenide mittels einem Tauch- oder Sprühverfahren aufgebracht ist. Beispielsweise kann eine pulverisierte Form von langkettigem, wasserunlöslichem Natriumpolyphosphat mit MgO trocken gemischt und dann in aufgeschlämmter Form durch Tauchen oder Sprühen auf die Endoprothese aufgebracht werden. Durch gleichzeitiges Besprühen mit oder nach dem Trocknen durch Tauchen in eine(r) konzentrierte(n), Magnesiumjodid und -chlorid enthaltende(n) wässrige Lösung kann eine Sorelzementschicht erzeugt werden. Dieser Vorgang kann solange wiederholt werden, bis eine Schichtdicke von etwa 20 µm bis etwa 30 µm erreicht ist. Anschließend kann durch Phosphatierung und lonentausch eine oberflächliche Erniedrigung der Löslichkeit und des pH-Werts in Richtung des Neutralbereichs erreicht werden.

Ebenso ist es bevorzugt, wenn die Beschichtung mittels einer Behandlung mit einer Alkali-/Erdalkalihalogenid- oder -hydroxidmischlösung hergestellt ist. Hierdurch kann in situ eine Sorelzementschicht erzeugt werden.

In vorteilhafter Weise kann die Löslichkeit der Oberfläche der Beschichtung verringert werden, in dem die Oberfläche zusätzlich mit einer Phosphorsäure- und/oder Phosphat-Lösung und/oder mit einer wasserabweisenden Versiegelung, vorzugsweise einem Wachs und/oder einem Fett, behandelt wird. Hierbei weisen die Phosphorsäure- und/oder Phosphat-Lösungen einen pH-Wert im neutralen oder leicht sauren (Phosphatpuffer-)Bereich auf. Bevorzugt werden Mischungen von Alkalihydrogen- und Alkalidihydrogenphosphaten verwendet.

Ein besonders vorteilhaftes Verfahren zur Herstellung einer intraluminalen Endoprothese besteht darin, dass der Grundkörper der Endoprothese mit unlöslichem Polyphosphatpulver beschichtet wird und dieses anschließend oberflächlich angeschmolzen wird, z.B. durch LASER-Bearbeitung. Mittels des erfindungsgemäßen Verfahrens lässt sich die Beschichtung einfach aufbringen. Durch das oberflächliche Anschmelzen des Pulvers wird eine dichte Polyphosphatschmelze ausgebildet.

Weitere Ziele, Merkmale, Vorteile und Anwendungsmöglichkeiten der Erfindung ergeben sich aus der nachfolgenden Beschreibung einer erfindungsgemäßen intraluminalen Endoprothese sowie eines Herstellungsverfahrens für eine solche Endoprothese anhand von Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den einzelnen Ansprüchen oder deren Rückbeziehung.

Es zeigen schematisch:
- Figur 1: einen Querschnitt eines extrudierten Rohrs, das ein Halbzeug zur Herstellung einer erfindungsgemäßen Endoprothese darstellt und
- Figuren 2 bis 3: einen Querschnitt einer ersten Spritzgussvorrichtung und
- Figur 4: einen Querschnitt einer zweiten Spritzgussvorrichtung, die zur Herstellung einer erfindungsgemäßen Endoprothese dienen.

### 1. Beispiel

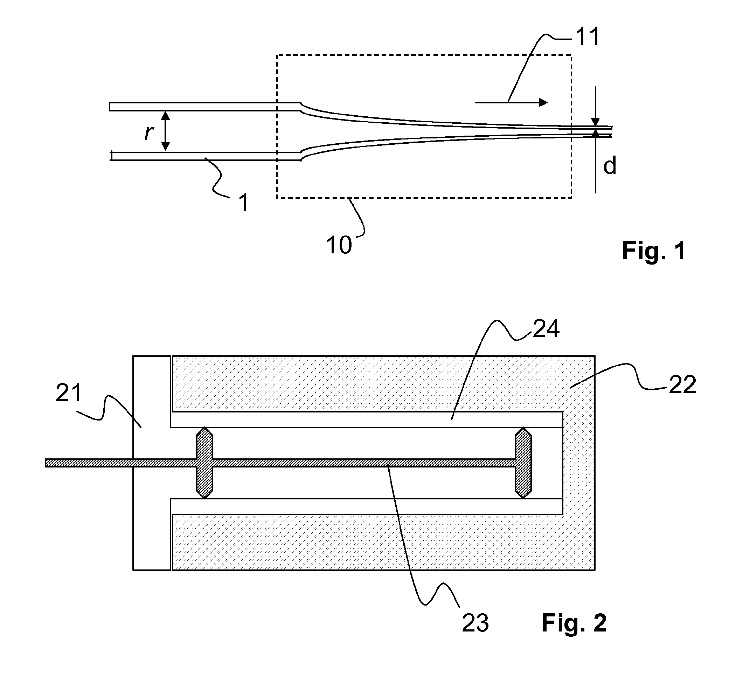

Zur Herstellung eines Stents wird zunächst aus einer Mischung von pulverisiertem Polyphosphat und Zuschlägen mit der Zusammensetzung von etwa 87 Gew.% wasserunlösliches Phosphatglas mit einem P₂O₅-Gehalt von etwa 65-70 Gew.% und 13 Gew.% Silberjodid eine Schmelze hergestellt und das in Figur 1 im Querschnitt dargestellte dünnwandige Rohr 1 mit einem Innendurchmesser r von beispielsweise etwa 5 mm und einer Wandstärke von 0,7 mm extrudiert. Dieses Rohr kann bei Bedarf unter geeigneter Temperaturführung, z.B. durch Erwärmen über einer Bunsenbrennerflamme bis zur Ziehfähigkeit, weiter zu einem dünnwandigeren Rohr geeigneter Wandstärke d, z.B. einer Wandstärke d von etwa 150 µm, von Hand oder in einer Vorrichtung 10 ausgezogen werden. Die Zugrichtung 11 in der Zugvorrichtung 10 verläuft in Richtung der Längsachse des Rohrs. Entsprechend der für das Rohrmaterial typischen Eigenschaften wird ein passendes Stentschnittmuster (Design) mit Hilfe eines auf der Methode der Finiten Elemente basierenden Rechnerprogramms entworfen. Mittels eines LASERs wird aus dem dünnwandigen Phosphatrohr ein Stentgrundkörper geschnitten und durch Politur geglättet. Nachfolgend wird bei Bedarf eine lonenaustauschprozedur angeschlossen.

### 2. Beispiel

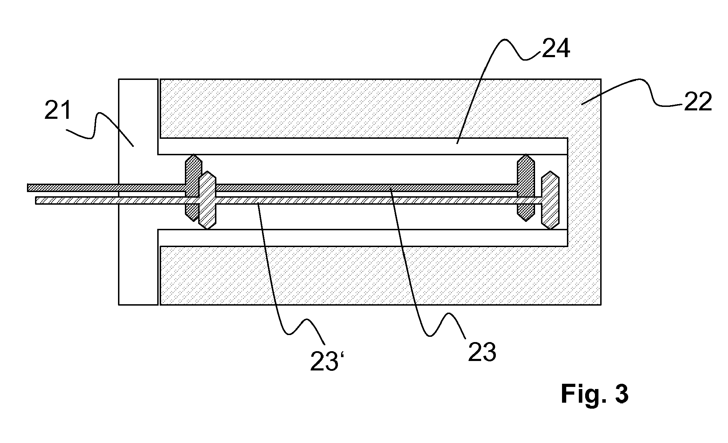

In einer geeigneten Spritzgussvorrichtung mit den ineinander gesteckten Formteilen 21 und 22, welche in den Figuren 2 und 3 dargestellt sind, wird aus einer erfindungsgemäßen wasserarmen Sorelzementmischung ein dünnwandiges Rohr (Hohlzylinder) geformt. Der Hohlzylinder wird dabei durch den hohlzylinderförmigen Hohlformbereich 24 ausgebildet, welcher zwischen dem hohlzylinderförmigen (mit Boden) Formteil 22 und dem Doppelzylinderförmigen Formteil 21 erzeugt wird. Die Sorelzementmischung ist zusammen mit Zuschlägen enthaltend Polyphosphate und/oder anderen radioopaken und/oder pharmazeutisch aktive und/oder die mechanischen Eigenschaften verbessernden Substanzen, wie etwa in Biopolymere eingebettete pharmakologisch aktive Substanzen der weiter oben genannten Substanzklassen, in dem Hohlformbereich 24 angeordnet. Durch Einspritzen von Wasser (H₂O) und/oder einer Lösung von Zuschlägen (z.B. MgCl₂-Lösung) in ein oder mehrere Spritzkanalsystem(e) (Bezugszeichen 23 und 23' in den Figuren 2 bis 3) werden dem eventuell aus Trockensubstanz bestehenden Rohr Zuschläge zugemischt oder der Aushärtungsvorgang eingeleitet. Das ausgehärtete Rohr wird anschließend analog zum Rohr des ersten Beispiels einer Stentschnittprozedur unterworfen. Danach kann wiederum eine Politur, eine Phosphatierung und/oder eine weitere Beladung mit pharmakologisch wirksamen oder die Degradation steuernden Substanzen erfolgen, wie etwa eine Imprägnierung mit wasserunlöslichen oder pharmakologisch aktiven Lipiden. Ferner kann sich nach der Formgebung oder nach der Schnittprozedur eine thermische Behandlung, wie etwa eine Erhitzung auf etwa 45°C bis etwa 450 °C anschließen, um ein Sintern oder eine Homogenisierung des Materials zu erreichen.

### 3. Beispiel

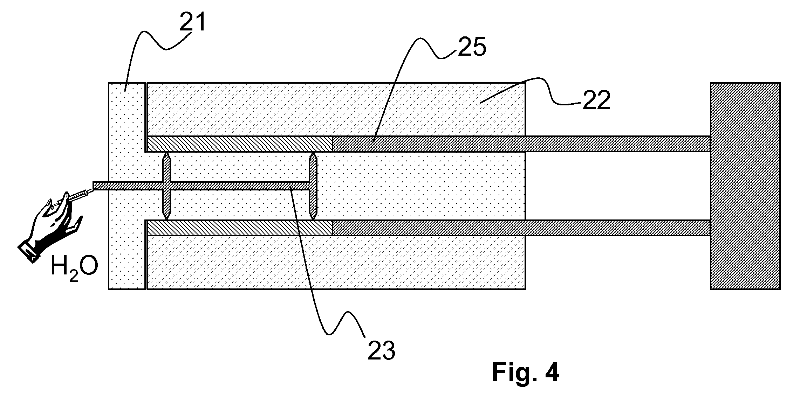

In einer Vorrichtung gemäß Figur 4 wird ein homogenisiertes Gemisch von pulverförmigen, wasserarmen Bestanteilen mit erfindungsgemäßer Stöchiometrie bestehend z.B. aus etwa 55 Gew.% einer MgO-, MgCl₂- (wasserfrei), MgJ₂- (wasserfrei)-Mischung entsprechend Formel 1 und einem wasserunlöslichen Polyphosphat mit einem P₂O₅-Gehalt von etwa 70 Gew.% mit Hilfe des Rohrstempels 25 in dem rohrförmigen Hohlraum 24, der aus dem Formen 21 und 22 ausgebildet wird, mit einem Druck von etwa 100 kg/cm² (oder mehr) verdichtet und/oder bei Temperaturen bis etwa 450°C gesintert und anschließend mit gereinigtem Wasser oder einer Salzlösung zur Sorelzementbildung versetzt oder durch nachträgliche Erhitzung und/oder Befeuchtung zu einem stabilen Stentgrundkörper verfestigt. Die Spritzgussvorrichtung in Figur 4 entspricht in ihrem Aufbau bis auf den Rohrstempel 25 der in den Figuren 2 und 3 gezeigten Vorrichtung.

### Bezugszeichenliste

- 1: Rohr
- 10: Zugvorrichtung
- 11: Zugrichtung
- 21, 22: Spritzgussform, z.B. aus Metall
- 23, 23': Einspritzkanal mit endständigen Einspritzdüsen
- 24: Hohlformbereich
- 25: rohrförmiger Druckstempel zur Verpressung des Materials im Formbereich 24
- d: Wandstärke des gezogenen Rohrs 1
- r: Innendurchmesser des extrudierten Rohrs 1

## Patentansprüche

1. Intraluminale Endoprothese, vorzugsweise ein Stent, bestehend aus einem Grundkörper und ggf. einer die Oberfläche des Grundkörpers mindestens teilweise bedeckenden Beschichtung, **dadurch gekennzeichnet, dass** der Grundkörper und/oder die Beschichtung mindestens eine Verbindung aus der Gruppe
Polyphosphate, Magnesiumoxidhalogenide, vorzugsweise Sorelzement, aufweist.

2. Intraluminale Endoprothese nach Anspruch 1, **dadurch gekennzeichnet, dass** der Grundkörper und/oder die Beschichtung mit mindestens einer pharmazeutisch aktiven Substanz und/oder einer röntgenopaken Substanz, vorzugsweise mindestens einem Element oder einer Verbindung der Gruppe
Biopolymere, vorzugsweise Proteine, Peptide, Aminosäuren, Nucleinsäuren, Polysaccharide,
Lipide, vorzugsweise Fettsäuren, Fettsäureester, Wachse,
hydrophobe Verbindungen, vorzugsweise Cholesterinester, Phosphatester, Kieselsäureethylester,
röntgenopake Elemente oder Verbindungen, vorzugsweise Edelmetalle, Metallsalze, Jodverbindungen,
versehen ist.

3. Intraluminale Endoprothese nach einem der Ansprüche 1 bis 2, **dadurch gekennzeichnet, dass** der Grundkörper und/oder die Beschichtung Sorelzement mit einem MgO-Unter- oder-Überschuss aufweist.

4. Intraluminale Endoprothese nach einem der Ansprüche 1 bis 3, **dadurch gekennzeichnet, dass** der Grundkörper und/oder die Beschichtung ein Magnesiumoxidhalogenid aufweist mit
[(1-a) MgCl₂ a MgJ₂] x b Mg(OH)₂ x c H₂O, mit 0 < a ≤ 1,1 < b ≤ 7, c > 1.

5. Intraluminale Endoprothese nach einem der Ansprüche 1 bis 4, **dadurch gekennzeichnet, dass** die Endoprothese einen Grundkörper, der eine Metalllegierung enthält, und eine Beschichtung aufweist, die Sorelzement enthält, wobei die Beschichtung eine Schichtdicke von mindestens etwa 5 µm, vorzugsweise mindestens etwa 50 µm und maximal etwa 200 µm hat.

6. Intraluminale Endoprothese nach einem der Ansprüche 1 bis 5, **dadurch gekennzeichnet, dass** die Endoprothese mindestens teilweise mit einer Polyphosphat-Beschichtung versehen ist, die eine Schichtdicke von mindestens etwa 5 µm, vorzugsweise mindestens etwa 30 µm und maximal etwa 200 µm aufweist, wobei die Moleküle der Polyphosphat-Beschichtung vorzugsweise eine Kettenlänge von mindestens 10 Phosphatgruppen aufweisen.

7. Intraluminale Endoprothese nach einem der Ansprüche 1 bis 6, **dadurch gekennzeichnet, dass** der Grundkörper und/oder die Beschichtung mindestens etwa 30 Gew.% P₂O₅, das ein Polyphosphat ausbildet, aufweist, bezogen auf den wasserfreien Mineralgehalt der Grundkörpers und/oder der Beschichtung.

8. Intraluminale Endoprothese nach einem der Ansprüche 1 bis 7, **dadurch gekennzeichnet, dass** die Beschichtung mittels einem Tauch- oder Sprühverfahren aufgebracht ist.

9. Intraluminale Endoprothese nach einem der Ansprüche 1 bis 8, **dadurch gekennzeichnet, dass** die Magnesiumoxidhalogenid-Beschichtung mittels einer Behandlung mit einer Alkali-/Erdalkalihalogenid- oder einer -hydroxidmischlösung hergestellt ist.

10. Intraluminale Endoprothese nach einem der Ansprüche 1 bis 9, **dadurch gekennzeichnet, dass** die Oberfläche der Magnesiumoxidhalogenid-Beschichtung zusätzlich mit einer Phosphorsäure- und/oder Phosphat-Lösung und/oder mit einer wasserabweisenden Versiegelung, vorzugsweise mit einem Wachs und/oder einer anderen lipophilen Substanz, behandelt ist.

11. Verfahren zur Herstellung einer intraluminalen Endoprothese nach Anspruch 1, **dadurch gekennzeichnet, dass** der Grundkörper mindestens teilweise mit unlöslichem Polyphosphatpulver beschichtet wird, vorzugsweise mittels eines Tauch- oder Sprühverfahrens, und dieses anschließend oberflächlich angeschmolzen wird.

## Claims

1. An intraluminal endoprosthesis, preferably a stent, comprising a base body and optionally comprising a coating at least partially covering the surface of the base body, **characterized in that** the base body and/or the coating contains at least one compound from the group
polyphosphate, magnesium oxide halogenide, preferably magnesia cement.

2. The intraluminal endoprosthesis according to claim 1, **characterized in that** the base body and/or the coating is provided with at least one pharmaceutically active substance and/or an x-ray opaque substance, preferably at least one element or a compound from the group
biopolymers, preferably proteins, peptides, amino acids, nucleic acids, polysaccharides,
lipids, preferably fatty acids, fatty acid esters, waxes,
hydrophobic compounds, preferably cholesterol esters, phosphate esters, silicic acid esters,
x-ray opaque elements or compounds, preferably noble metals, metal salts, iodine compounds.

3. The intraluminal endoprosthesis according to one of the claims 1 to 2, **characterized in that** the base body and/or the coating comprises magnesia cement with an MgO deficiency or surplus.

4. The intraluminal endoprosthesis according to any one of the claims 1 to 3, **characterized in that** the base body and/or the coating comprises a magnesium oxide halogenide with
[(1-a) MgCl₂ a MgJ_{2]} x b Mg(OH)₂ x c H₂O, with 0 < a ≤ 1, 1 < b ≤ 7, c > 1.

5. The intraluminal endoprosthesis according to any one of the claims 1 to 4, **characterized in that** the endoprosthesis comprises a base body, which contains a metal alloy, and a coating, which contains magnesia cement, wherein the coating has a layer thickness of at least approximately 5 µm, preferably at least approximately 50 µm and, at most, approximately 200 µm

6. The intraluminal endoprosthesis according to any one of the claims 1 to 5, **characterized in that** the endoprosthesis is provided, at least in part, with a polyphosphate coating, which has a layer thickness of at least approximately 5 µm, preferably at least approximately 30 µm and, at most, approximately 200 µm, wherein the molecules of the polyphosphate coating preferably have a chain length of at least 10 phosphate groups.

7. The intraluminal endoprosthesis according to any one of the claims 1 to 6, **characterized in that** the base body and/or the coating comprises at least approximately 30% by weight of P₂O₅, which forms a polyphosphate, relative to the anhydrous mineral content of the base body and/or the coating.

8. The intraluminal endoprosthesis according to any one of the claims 1 to 7, **characterized in that** the coating is applied by means of an immersion or aerosol mist procedure.

9. The intraluminal endoprosthesis according to any one of the claims 1 to 8, **characterized in that** the magnesium oxide halogenide coating is produced by means of a treatment with a mixed solution of alkaline/alkaline earth halogenide or alkaline/alkaline earth hydroxide.

10. The intraluminal endoprosthesis according to any one of the claims 1 to 9, **characterized in that** the surface of the magnesium oxide halogenide coating is additionally treated with a phosphoric acid solution and/or a phosphate solution and/or with a water-repellent seal, preferably with a wax and/or another lipophilic substance.

11. A method for the manufacture of an intraluminal endoprosthesis according to claim 1, **characterized in that** the base body is coated, at least in part, with insoluble polyphosphate powder, preferably by means of an immersion or spray coat procedure, and this is subsequently melted surficially.

## Revendications

1. Endoprothèse intraluminale, de préférence un stent, constituée d'un corps de base et éventuellement d'un revêtement couvrant au moins partiellement la surface du corps de base, **caractérisée en ce que** le corps de base et/ou le revêtement comporte un composé parmi le groupe
des polyphosphates, des halogénures d'oxyde de magnésium, de préférence du ciment de Sorel.

2. Endoprothèse intraluminale selon la revendication 1, **caractérisée en ce que** le corps de base et/ou le revêtement est pourvu d'au moins une substance active pharmaceutique et/ou d'une substance radio-opaque, de préférence d'au moins un élément ou composé parmi le groupe
des biopolymères, de préférence des protéines, des peptides, des acides aminés, des acides nucléiques, des polysaccharides,
des lipides, de préférence des acides gras, des esters d'acides gras, des cires, des composés hydrophobes, de préférence des esters de cholestérol, des esters de phosphate, des esters éthyliques d'acides siliciques,
des éléments ou des composés radio-opaques, de préférence des métaux nobles, des sels métalliques, des composés d'iode.

3. Endoprothèse intraluminale selon l'une des revendications 1 à 2, **caractérisée en ce que** le corps de base et/ou le revêtement comporte du ciment de Sorel avec une insuffisance ou un excédent de MgO.

4. Endoprothèse intraluminale selon l'une des revendications 1 à 3, **caractérisée en ce que** le corps de base et/ou le revêtement comporte un halogénure d'oxyde de magnésium, avec
[(1-a) MgCl₂ a M_{g}J₂] x b Mg(OH)₂ x c H₂O, avec 0 < a ≤ 1, 1 < b ≤ 7, c > 1.

5. Endoprothèse intraluminale selon l'une des revendications 1 à 4, **caractérisée en ce que** l'endoprothèse comporte un corps de base contenant un alliage de métaux, ainsi qu'un revêtement contenant du ciment de Sorel, sachant que le revêtement présente une épaisseur de couche d'au moins environ 5 µm, de préférence d'au moins environ 50 µm et au maximum d'environ 200 µm.

6. Endoprothèse intraluminale selon l'une des revendications 1 à 5, **caractérisée en ce que** l'endoprothèse est au moins partiellement pourvue d'un revêtement de polyphosphate, présentant une épaisseur de couche d'au moins environ 5 µm, de préférence d'au moins environ 30 µm et de maximum environ 200 µm, sachant que les molécules du revêtement de polyphosphate présentent de préférence une longueur de chaîne d'au moins 10 groupes phosphates.

7. Endoprothèse intraluminale selon l'une des revendications 1 à 6, **caractérisée en ce que** le corps de base et/ou le revêtement comporte au moins environ 30% en poids de P₂O₅, constituant un polyphosphate, par rapport à la teneur en minéraux anhydres du corps de base et/ou du revêtement.

8. Endoprothèse intraluminale selon l'une des revendications 1 à 7, **caractérisée en ce que** le revêtement est appliqué au moyen d'un procédé d'immersion ou de pulvérisation.

9. Endoprothèse intraluminale selon l'une des revendications 1 à 8, **caractérisée en ce que** le revêtement d'halogénure d'oxyde de magnésium est fabriqué au moyen d'un traitement avec une solution mixte d'halogénures ou d'hydroxydes alcalins/alcalino-terreux.

10. Endoprothèse intraluminale selon l'une des revendications 1 à 9, **caractérisée en ce que** la surface du revêtement d'halogénure d'oxyde de magnésium est en outre traitée avec une solution d'acide phosphorique et/ou de phosphates et/ou avec un scellant hydrophobe, de préférence avec une cire et/ou une autre substance lipophile.

11. Procédé pour la fabrication d'une endoprothèse intraluminale selon la revendication 1, **caractérisé en ce que** le corps de base est au moins partiellement revêtu d'une poudre de polyphosphate insoluble, de préférence au moyen d'un procédé d'immersion ou de pulvérisation, et **en ce que** celle-ci est ensuite fondue superficiellement.
